# EUROPEAN PATENT APPLICATION

(11) **EP 3 730 156 A1**
(43) Date of publication of application: **28.10.2020**
(21) Application number: 19305532.4
(22) Date of filing: 25.04.2019
(51) Int. Cl.: A61L 2/20

(54) **TREATMENT MACHINE FOR TREATING RECEPTACLES AND METHOD OF OPERATING SUCH A TREATMENT MACHINE**

(71) Applicant: Sidel Participations, 76930 Octeville-sur-Mer (FR)
(72) Inventor: LETELLIER, Sandy, 76930 Octeville-sur-mer (FR); LIAO, Owen, Beijing, 100176 (CN); SU, Cheng, Shanghai, 200131 (CN); LICHNEWSKY, Lionel, 43126 Parma (IT)
(74) Representative: Sidel Group

(57) **Abstract**

There is described a treatment machine (1) for executing at least one treatment on a receptacle (2, 3). The treatment machine (1) comprises at least a control unit (5) configured to control operation of the treatment machine (1) and an active agent conditioning device (30) configured to condition a cleaning and/or sterilization mixture comprising at least hydrogen peroxide. The active agent conditioning device (30) is configured to at least control the temperature of the cleaning and/or sterilization mixture. The control unit (5) is configured to control the active agent conditioning device (30) into at least a sterilizing configuration and a cleaning configuration. The active agent conditioning device (30) is configured to control the temperature of the cleaning and/or sterilization mixture to be above a threshold temperature when being controlled into the sterilizing configuration and to control the temperature of the cleaning and/or sterilization mixture to be below a threshold temperature, when being controlled into the cleaning configuration.

## Description

### TECHNICAL FIELD

The present invention relates to a treatment machine for the treatment of receptacles such as bottles and/or preforms, in particular for the treatment of receptacles under aseptic conditions.

Advantageously, the present invention also relates to a method of operating a treatment machine for the treatment of receptacles, in particular for the treatment of receptacles such as bottles and/or bottle preforms under aseptic conditions.

### BACKGROUND ART

There are known treatment machines for the treatment of receptacles such as bottles, preforms, containers or the like, in particular for the treatment of the receptacles under sterile conditions.

For example, there are known treatment machines, which are configured to produce the receptacles (e.g. bottles) from another kind of receptacle (e.g. preforms), to sterilize the receptacles (bottles and/or preforms), to fill the receptacles and/or to close the receptacles.

It is also known that such treatment machines often comprise an isolation housing configured to allow for the treatment of the receptacles within a controlled atmosphere.

In particular, such kind of treatment apparatuses are known in the field of packaging pourable products into receptacles such as bottles.

Such a typical treatment machine comprises at least:
- a filling apparatus configured to fill the bottles with a pourable product; and/or
- a capping apparatus configured to apply closures onto the bottles; and/or
- a molding apparatus configured to mold the bottles from preforms; and/or
- a preparation apparatus configured to prepare the preforms.

It is also known to control the conditions under which the treatment of the receptacles should be executed, in particular so as to control the load of contaminations such as bacteria, microbes, spores and similar. Therefore, treatment machines may also comprise a sterilization apparatus for sterilizing portions of the treatment machine and/or of the receptacles by means of a chemical active agent.

One of the most commonly used cleaning and/or sterilizing apparatuses uses hydrogen peroxide as the active agent.

The hydrogen peroxide is provided within a sterilization mixture containing hydrogen peroxide, air and stabilizing agents.

The sterilizing apparatus also comprises a conditioning unit configured to control at least the temperature of the sterilization mixture to be at least 110 °C, in a significant number of applications being at least 130 °C.

However, it has been observed that the needed stabilizing agents lead to the deposition of solid residues on the surfaces of conducts of the sterilizing apparatus and/or onto other surfaces and/or ducts of the treatment machine. Over time, the solid residues need to be removed in order to guarantee the correct sterilization, which requires mechanically dismantling the conducts and/or other parts of the treatment machine followed by the rinsing and reassembling of these parts.

Thus, overall, the removal of the solid residues requires a significant time during which the treatment of the receptacles needs to be interrupted, leading also to overall increased production costs.

Hence, the desire is felt in the sector to reduce the downtime of the treatment machines due to the need of removing the solid residues originating from the needed sterilization.

Therefore, it is the desire to further improve the known treatment machines.

In particular, it is a desire to reduce the downtimes of the treatment machine as a consequence the needed cleaning so as to remove residues originating from the sterilization processes.

In particular, it is also a desire to further improve the known methods of operating the treatment machines, even more particular for reducing any needed downtimes of the treatment machines.

### DISCLOSURE OF INVENTION

It is therefore an object of the present invention to provide in a straightforward and low-cost manner a treatment machine for overcoming at least one of the above-mentioned drawbacks.

It is another object of the present invention to provide in a straightforward and low-cost manner a method of operating a treatment machine for overcoming at least one of the above-mentioned drawbacks.

According to the present invention, there is provided a treatment machine and methods according to the respective independent claims.

Further advantageous embodiments are specified in the respective claims being directly and/or indirectly dependent on the independent claims.

### BRIEF DESCRIPTION OF THE DRAWINGS

A non-limiting embodiment of the present invention will be described by way of example with reference to the accompanying drawings, in which:
Figure 1 is a schematic representation of a first portion of a treatment machine according to the present invention, with parts removed for clarity;
Figure 2 is a schematic representation of a second portion of the treatment machine according to the present invention with the second portion being immediately downstream of the first portion, with parts removed for clarity; and
Figure 3 is an enlarged schematic view of a detail of the treatment machine of Figure 1, with parts removed for clarity.

### BEST MODES FOR CARRYING OUT THE INVENTION

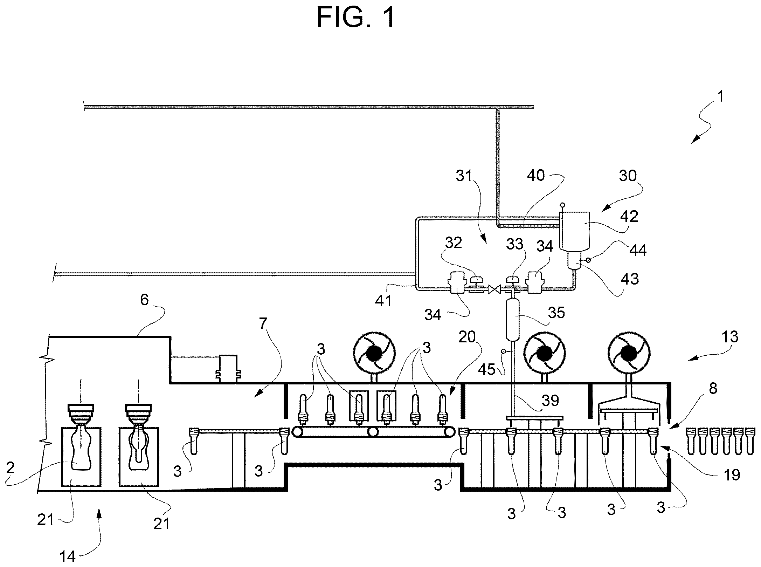

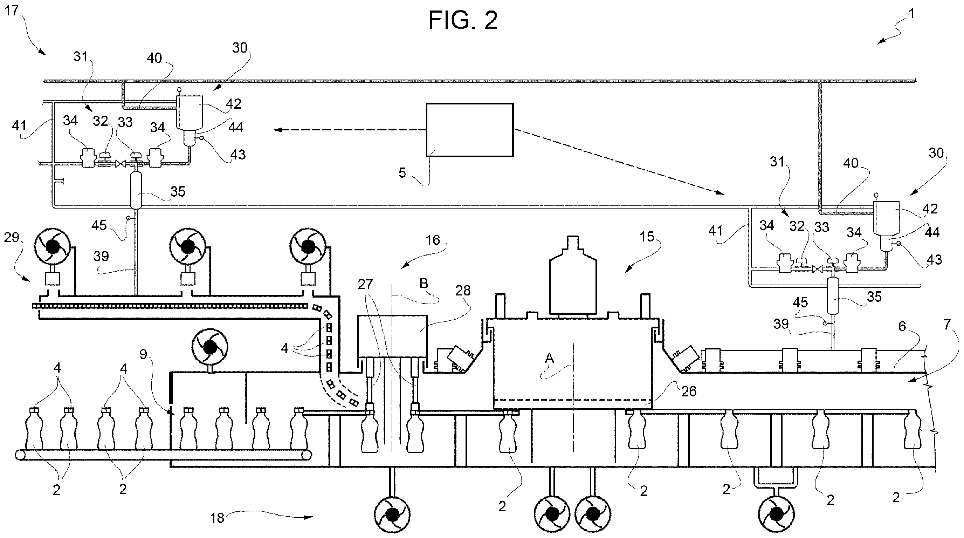

With particular reference to Figures 1 and 2, reference number 1 indicates as a whole a treatment machine for the treatment of receptacles, in particular for the treatment of receptacles under controlled and/or sterile conditions.

The receptacles can be of any type including bottles 2, preforms 3, jars, vessels, containers or the like, in particular being made of base components, like glass, paper or cardboard, plastics, aluminum, steel, and composites.

In particular, the receptacles may be adapted to be filled with a pourable product, in particular a pourable food product, even more particular a liquid or a viscous pourable food product.

In the following, we limit the description to the specific example of bottles 2 adapted to be filled with any type of pourable food product such as carbonated liquids (e.g. sparkling water, soft drinks and beer), non-carbonated liquids (including still water, juices, teas, sport drinks, wine, etc.) and beverages containing pulps. However, it should be clear that the invention also relates to other types of receptacles, such as preforms, containers or the like. It should also be clear that the invention also relates to receptacles, which are adapted to be filled with other kinds of pourable products such as sugar, salt and others.

According to a preferred non-limiting embodiment shown, treatment machine 1 is configured to treat more than one kind of receptacle.

In the non-limiting example shown, treatment machine 1 is configured to treat bottles 2 and preforms 3. In particular, preforms 3 define a precursor of bottles 2. In other words, bottles 2 are obtained from preforms 3.

According to alternative non-limiting embodiments, treatment machine 1 could be configured to treat bottles 2 only or preforms 3 only.

According to a preferred non-limiting embodiment, treatment machine 1 is configured to at least:
- fill bottles 2 with the pourable product; and/or
- apply closures 4 onto bottles 2; and/or
- sterilize bottles 2 and/or preforms 3 and/or closures 4; and/or
- sterilize portions of treatment machine 1; and/or
- form bottles 2 from preforms 3; and/or
- prepare preforms 3 for being formed into bottles 2.

Advantageously, treatment machine 1 comprises a control unit 5 configured to control operation of treatment machine 1. In particular, control unit 5 is also configured to control the treatment of bottles 2 and/or preforms 3.

According to a preferred non-limiting embodiment, treatment machine 1 comprises at least an isolation housing 6 so as to treat the receptacles, in particular bottles 2 and/or preforms 3, within a (controlled) inner environment 7 and/or under a controlled atmosphere.

Preferentially but not necessarily, isolation housing 6 is configured to be arranged on a (substantially horizontal) surface (e.g. a floor of a production site). Preferentially but not necessarily, isolation housing 6 comprises an inlet opening 8 and an outlet opening 9, in particular for allowing respectively the inlet of preforms 3 into and the outlet of bottles 2, in particular bottles 2 being filled with the pourable product and being provided with respective closures 4, from inner environment 7.

According to a preferred non-limiting embodiment, treatment machine 1 comprises a plurality of treatment apparatuses, in particular being at least partially arranged within isolation housing 6, and each one configured to execute a distinct treatment on bottles 2 and/or preforms 3.

According to a preferred non-limiting embodiment, treatment machine 1 also comprises conveying means configured to advance bottles 2 along a bottle advancement path and/or preforms along a preform advancement path. Preferentially but not necessarily, each treatment apparatus comprises at least a portion of the conveying means.

According to a preferred non-limiting embodiment, the treatment apparatuses are chosen from the group of:
- a preform preparation apparatus 13 configured to prepare preforms 3 for being transformed into bottles 2, in particular during advancement along a preparation portion of the preform advancement path;
- a molding apparatus 14 configured to mold, in particular blow or compression mold, preforms 3 into bottles 2, in particular during advancement along a molding portion of the preform advancement path;
- a filling apparatus 15 configured to fill bottles 2 with the pourable product, in particular during advancement of bottles 2 along a filling portion of the bottle advancement path; and/or
- a capping apparatus 16 configured to apply closures 4 onto (filled) bottles 2, in particular during advancement of bottles 2 along a capping portion of the bottle advancement path.

Preferentially but not necessarily, treatment machine 1 comprises preform preparation apparatus 13 and/or molding apparatus 14 and/or filling apparatus 15 and/or capping apparatus 16.

Preferentially but not necessarily, preform preparation apparatus 13 is arranged upstream from molding apparatus 14 along the preform advancement path and is configured to transfer preforms 3 to molding apparatus 14.

Preferentially but not necessarily, filling apparatus 15 is arranged upstream from capping apparatus 16 along the bottle advancement path and is configured to transfer filled bottles 2 to capping apparatus 16.

Preferentially but not necessarily, molding apparatus 14 is arranged upstream from filling apparatus 15 along the bottle advancement path and is configured to transferred bottles 2 (to be filled) to filling apparatus 15.

According to a preferred non-limiting embodiment, treatment machine 1 also comprises a sterilization and cleaning apparatus 17 configured to sterilize preforms 3 and/or bottles 2 and/or portions of treatment machine 1 and/or of sterilization and cleaning apparatus 17 itself and/or to clean at least portions, in particular surfaces, of sterilization and cleaning apparatus 17 itself and/or of treatment machine 1. In particular, sterilization and cleaning apparatus 17 is configured to sterilize and/or clean by means of supplying a sterilization and/or cleaning mixture having at least one active agent.

According to a preferred non-limiting embodiment, treatment machine 1 is controllable, in particular by means of control unit 5, into at least:
- a production configuration during which the receptacles, in particular bottles 2 and/or preforms 3, are treated including the sterilization of the receptacles, in particular preforms 3 and/or bottles 2, by means of sterilization and cleaning apparatus 17;
- an initial sterilization configuration during which portions, in particular surfaces, of sterilization and cleaning apparatus 17 and/or treatment machine 1 are sterilized by means of sterilization and cleaning apparatus 17; and
- an initial cleaning configuration during which portions, in particular surfaces, of sterilization and cleaning apparatus 17 and/or treatment machine 1 are cleaned by means of sterilization and cleaning apparatus 17.

It should be noted that sterilization means that the loads of contaminants such as bacteria, microbes, spores and the like are reduced and cleaning means that any macroscopic contamination is removed.

According to a preferred non-limiting embodiment, treatment machine 1 also comprises an atmosphere control unit 18 configured to control a gas atmosphere within isolation housing 6 and/or of inner environment 7.

Preferentially but not necessarily, atmosphere control unit 18 is configured to at least:
- introduce a sterile gas, in particular sterile air, into isolation housing 6; and/or
- to selectively and locally control a gas pressure within isolation housing 6; and/or
- to selectively and locally control a gas flow within isolation housing 6.

With particular reference to Figure 1, preform preparation apparatus 13 comprises a conveyor unit 19 configured to advance preforms 3 along the preform preparation portion and a pre-heat unit 20 configured to heat preforms 3 prior to being transferred to molding apparatus 14.

With particular reference to Figure 1, molding apparatus 14 comprises a plurality of molding units 21, each one configured to retain one respective preform 3 and to mold the respective preform 3 into a respective bottle 2 during advancement of the respective preform 3 along the molding portion, and to retain the formed bottles 2 prior to their transfer to filling apparatus 15.

Preferentially but not necessarily, molding apparatus 14 also comprises a first conveying carousel rotatable around a first rotation axis, in particular having a vertical orientation, carrying molding units 21 and being configured to advance preforms 3 along the molding portion and/or to advance bottles 2 after their formation and prior to their transfer to filling apparatus 15. In particular, the first conveying carousel defines a portion of the conveying means.

Preferentially but not necessarily, molding units 31 are peripherally arranged on conveying carousel 29 and equally spaced around the first rotation axis.

Preferentially but not necessarily, molding units 21 are arranged within inner environment 7.

With particular reference to Figure 2, filling apparatus 15 comprises a plurality of filling units (not shown and known as such), each one configured to retain and fill one respective bottle 2 with the pourable product during its advancement along the filling portion.

Preferentially but not necessarily, filling apparatus 15 also comprises a conveying carousel 26 rotatable around a rotation axis A, in particular having a vertical orientation, for advancing bottles 2 along the filling portion. In particular, conveying carousel 26 defines another portion of the conveying means.

Preferentially but not necessarily, the filling units are peripherally arranged on conveying carousel 26 and equally spaced around rotation axis A.

In particular, the filling units are arranged within inner space 15.

According to a preferred non-limiting embodiment, capping apparatus 16 is configured to apply at least one respective closure 4 onto each bottle 2, in particular closures 4 may be of the type known as crown corks, screw caps, sports caps, stoppers etc., and they may be produced from a variety of materials such as plastics, cork, metal, composites and others.

According to a preferred non-limiting embodiment, capping apparatus 16 comprises a plurality of capping units 27, each one configured to retain one respective (filled) bottle 2 and to apply one respective closure 4 onto the respective (filled) bottle 2 during its advancement along the capping portion.

Preferentially but not necessarily, capping apparatus 16 also comprises a conveying carousel 28 rotatable around a rotation axis B, in particular having a vertical orientation, for advancing (filled) bottles 2 along the capping portion. In particular, conveying carousel 28 defines a further portion of the conveying means.

Preferentially but not necessarily, capping units 27 are peripherally arranged on conveying carousel 28 and equally spaced around rotation axis B.

Preferentially but not necessarily, capping units 27 are arranged within inner environment 7.

According to a preferred non-limiting embodiment, capping apparatus 16 also comprises a closure supply unit 29 configured to supply closures 4 to capping units 27.

According to a preferred non-limiting embodiment, control unit 5 is configured to control operation of sterilization and cleaning apparatus 17.

With particular reference to Figures 1 and 2, sterilization and cleaning apparatus 17 comprises one or more active agent conditioning devices 30, each one configured to condition and/or prepare a cleaning and/or sterilization mixture comprising at least hydrogen peroxide.

In particular, each active agent conditioning device 30 is configured to control and/or prepare the cleaning and/or sterilization mixture such that according to the specific conditioning and/or preparation the cleaning and/or sterilization mixture acts, in use, as a cleaning agent or as a sterilizing agent. Even more particular, when acting, in use, as a sterilizing agent, the cleaning and/or sterilization mixture is adapted to reduce the load of contaminates such as bacteria, microbes, spores and/or similar, and when acting, in use, as a cleaning agent, the cleaning and/or sterilization mixture, is adapted to remove residues, in particular solid residues, even more particular residues resulting at least in part from the cleaning and/or sterilization mixture itself.

According to a preferred non-limiting embodiment, the cleaning and/or sterilization mixture also comprises at least a carrier gas, in particular air, and a stabilizing agent, the stabilizing agent being configured to stabilize the cleaning and/or sterilization mixture, in particular when being at temperatures above 100 °C.

According to a preferred non-limiting embodiment, at least one active agent conditioning device 30 is associated to preform preparation apparatus 13 (see Figure 1) and is configured to at least distribute the cleaning and/or sterilization mixture into preforms 3 for sterilizing preforms 3.

According to a preferred non-limiting embodiment, at least one active agent conditioning device 30 is associated to capping apparatus 16 (see Figure 2), in particular closure supply unit 29, and is configured to at least distribute the cleaning and/or sterilization mixture into capping apparatus 16, in particular closure supply unit 29, and/or onto closures 4 for sterilizing closures 4.

Preferentially but not necessarily, sterilization and cleaning apparatus 17 also comprises a further active agent conditioning device 30 configured to inject the cleaning and/or sterilization mixture into varying portions of treatment machine 1.

According to a preferred non-limiting embodiment, each active agent conditioning device 30 is also configured to condition the cleaning and/or sterilization mixture for sterilizing and/or to cleaning at least portions of the active agent conditioning device 30 itself and/or of treatment machine 1.

Advantageously, each active agent conditioning device 30 is configured to at least control the temperature of the respective cleaning and/or sterilization mixture.

Advantageously, control unit 5 is configured to (selectively) control each active agent conditioning device 30 into at least:
- a sterilizing configuration; and
- a cleaning configuration.

Advantageously, each active agent conditioning device 30 is configured to control the temperature of the respective cleaning and/or sterilization mixture to be above and below a threshold temperature when being controlled into respectively the respective sterilizing configuration and the respective cleaning configuration.

According to a preferred non-limiting embodiment, the threshold temperature is 100 °C and each active agent conditioning device 30 is configured to control the temperature of the respective cleaning and/or sterilization mixture to be above 100 °C, in particular to be at least 110 °C, even more particular to be at least 130 °C when being controlled into the respective sterilization configuration and to be below 100 °C, in particular to be below 70°C, when being controlled into the cleaning configuration.

It should be noted that according to the present invention, the temperature of the cleaning and/or sterilization mixture is the temperature of the cleaning and/or sterilization mixture as determined by the respective active agent conditioning device 30. The temperature of the cleaning and/or sterilization mixture may differ at the point of sterilization and/or cleaning. In other words, in use, the cleaning and/or sterilization mixture exiting from the respective active agent conditioning device 30 has a temperature, which is above and below the threshold temperature when the respective active agent conditioning device 30 is controlled respectively into the sterilization configuration and the cleaning configuration.

According to a preferred non-limiting embodiment, control unit 5 is configured to (selectively) control each active agent conditioning device 30:
- into the sterilization configuration so that, in use, the active agent conditioning device conditions the respective cleaning and/or sterilization mixture for sterilizing portions of treatment machine 1 and/or of bottles 2 and/or closures 4 and/or preforms 3; and/or
- into the cleaning configuration so that, in use, the active agent conditioning device conditions the respective cleaning and/or sterilization mixture for cleaning portions of the Treatment machine and/or of the active agent conditioning device, in particular for removing (solid) residues originating from the cleaning and/or sterilization mixture, in particular from the stabilizing agent.

In particular, the control of the cleaning and/or sterilization mixture to a temperature above the threshold temperature (e.g. above 100 °C) allows to support the sterilization activity of the cleaning and/or sterilization mixture, while the control of the cleaning and/or sterilization mixture to a temperature below the threshold temperature (e.g. below 100°C) allows to clean active agent conditioning device 30 and/or portions of treatment machine 1, in particular for removing (solid) residues present on surfaces of active agent conditioning device 30 and/or treatment machine 1, in particular the (solid) residues comprise at least partially the stabilizing agent.

Preferentially but not necessarily, each active agent conditioning device 30 is configured to control the temperature of the cleaning and/or sterilization mixture to range between 100 °C to 200 °C, in particular between 110 °C to 200 °C, even more particular between 130 °C to 200 °C.

Preferentially but not necessarily, each active agent conditioning device 30 is also configured to control at least the concentration of the hydrogen peroxide and/or the physical state (e.g. an aerosol or liquid etc.) of the respective cleaning and/or sterilization mixture.

Preferentially but not necessarily, each active agent conditioning device 30 is also configured to condition the respective cleaning and/or sterilization mixture such that the concentration of the hydrogen peroxide is above 2000 ppm (parts per million), in particular so that the concentration ranges between 2000 ppm (parts per million) to 100000 ppm.

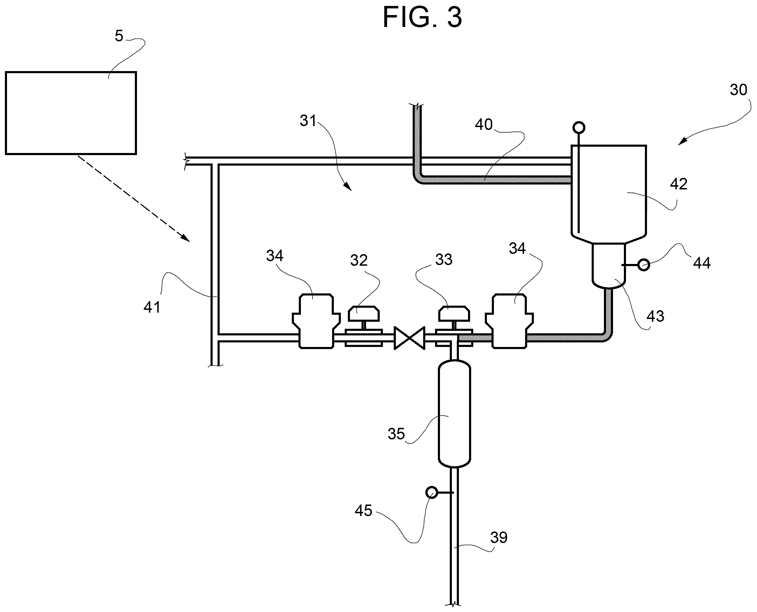

With particular reference to Figures 1 to 3, each active agent conditioning device 30 comprises a mixing assembly 31 configured to mix an initial hydrogen peroxide mixture with a carrier gas, in particular air, for obtaining the cleaning and/or sterilization mixture and so as to control the hydrogen peroxide concentration of the cleaning and/or sterilization mixture, in particular to range between 2000 ppm to 100000 ppm.

In particular, the hydrogen peroxide concentration of the initial hydrogen peroxide mixture is higher than the one of the cleaning and/or sterilization mixture.

According to a preferred non-limiting embodiment, each mixing assembly 31 is configured to mix pressurized air as the carrier gas with the initial hydrogen peroxide mixture.

In particular, each mixing assembly 31 comprises at least a first valve 32 for controlling a flow of the carrier gas and a second valve 33 for controlling a flow of the initial hydrogen peroxide mixture.

According to an alternative embodiment not shown, each mixing assembly 31 could comprise one valve element or a valve assembly providing for the function of valve 32 and 33.

Alternatively or in addition, each mixing assembly 31 could comprise one or more pumps for controlling the mixing of the carrier gas and the initial hydrogen peroxide mixture.

Preferentially but not necessarily, each mixing assembly 31 could also comprise two flow meters 34 one configured to determine and/or measure the flow of the carrier gas and the other one configured to determine and/or measure the flow of the initial hydrogen peroxide mixture.

According to a preferred non-limiting embodiment, each first valve 32 is controlled such that the flow of the carrier gas ranges between 5 1/min to 500 1/min; and the respective second valve 33 is controlled such that the flow of the initial hydrogen peroxide mixture ranges between 50 g/hour to 10000 g/hour.

According to a preferred non-limiting embodiment, each active agent conditioning device 30 also comprises a heating device, in particular an evaporator 35, configured to control and/or determine at least the temperature of the cleaning and/or sterilization mixture.

According to a preferred non-limiting embodiment, each heating device, in particular each evaporator 35, is configured to control the temperature of the cleaning and/or sterilization mixture to be above and below the threshold temperature (e.g the threshold temperature being 100°C) with the respective active agent conditioning device 30 being respectively controlled into the sterilization configuration and the cleaning configuration.

It should be noted that each heating device, in particular each evaporator 35, could, in particular with the respective active agent conditioning device 30 being controlled into the cleaning configuration, being deactivated as in some cases no active heating is required for obtaining the desired and/or required temperatures.

Preferentially but not necessarily, each heating device, in particular each evaporator 35, is configured to control the physical state of the cleaning and/or sterilizing mixture. In particular, each heating device is configured to control the cleaning and/or sterilizing mixture to be an aerosol.

According to a preferred non-limiting embodiment, each heating device, in particular each evaporator 35, is configured to receive the cleaning and/or sterilization mixture from the respective mixing assembly 31.

According to a preferred non-limiting embodiment, each active agent conditioning device 30 also comprises at least:
- a delivery tube 39 configured to direct the cleaning and/or sterilization mixture to treatment machine 1; and/or
- an inlet tube 40 for receiving hydrogen peroxide; and/or
- an inlet tube 41 for receiving the pressurized air.

Preferentially but not necessarily, delivery tube 39 is connected to the respective heating device and is configured to receive the cleaning and/or sterilizing agent from the respective heating device.

According to a preferred non-limiting embodiment, when controlling the respective active agent conditioning unit 30 into the respective cleaning configuration, the cleaning and/or sterilization mixture also cleans delivery tube 39 and/or at least portions of the respective heating device.

According to a preferred non-limiting embodiment, each active agent conditioning device 30 also comprises a storage tank 42 for storing and providing the initial hydrogen peroxide mixture, in particular being connected to the respective inlet tube 40.

Preferentially but not necessarily, each active agent conditioning device 30 also comprises at least one filter element 43 configured to filter the initial hydrogen peroxide mixture.

According to a preferred non-limiting embodiment, each active agent conditioning device 30 also comprises a pressure sensor 44.

According to a preferred non-limiting embodiment, each active agent conditioning device 30 also comprises a temperature sensor 45 configured to measure the temperature of the respective cleaning and/or sterilizing mixture, in particular after operation of the respective heating device.

In use, treatment machine 1 treats the receptacles, in particular bottles 2 and/or preforms 3 when being controlled into the production configuration.

According to a preferred non-limiting embodiment, treatment machine 1 prepares preforms 3 and/or molds preforms 3 into bottles 2, and/or sterilizes preforms 3 and/or fills bottles 2 with the pourable product and/or applies closures 4 onto bottles 2, in particular when being controlled into the production configuration.

According to a preferred non-limiting embodiment, with treatment machine 1 being controlled into the production configuration, preform preparation apparatus 13 prepares preforms 3 during advancement of preforms 3 along the preform preparation portion, in particular pre-heat unit 20 heats preforms 3; and/or preform preparation apparatus 13 transfers preforms 3 to molding apparatus 14; and/or molding apparatus 14 molds preforms 3 into bottles 2 during advancement of preforms 3 along the molding portion; and/or transferring bottles 2 from molding apparatus 14 to filling apparatus 15; and/or filling apparatus 15 filling bottles 2 with the pourable product during advancement of bottles 2 along the filling portion; and/or filling apparatus 15 transferring (the filled) bottles 2 to capping apparatus 16; and/or capping apparatus 16 applying closures 4 onto bottles 2 during advancement of bottles 2 along the capping portion.

According to a preferred non-limiting embodiment, during operation of treatment machine 1, in particular when being controlled into the production configuration, atmosphere control unit 18 controls the gas atmosphere within isolation housing 6 and/or inner environment 7, in particular for controlling a sterile gas atmosphere and/or for selectively and locally controlling the gas pressure and/or the gas flow.

Operation of treatment machine 1 also comprises the step of sterilizing the receptacles, in particular bottles 2 and/or preforms 3, and/or closures 4 and/or portions, in particular surfaces, of treatment machine 1 and/or sterilizing and cleaning apparatus 17 and/or active agent conditioning devices 30 by means of the (respective) cleaning and/or sterilization mixture.

In particular, the step of sterilizing is executed for sterilizing the receptacles, in particular bottles 2 and/or preforms 3, and/or closures 4 while treatment machine 1 is controlled into the production configuration.

In particular, the step of sterilizing is executed for sterilizing portions, in particular surfaces, of treatment machine 1 and/or sterilizing and cleaning apparatus 17 and/or active agent conditioning devices 30 while treatment machine 1 is controlled into the initial sterilization configuration.

Operation of treatment machine 1 also comprises the step of cleaning, during which portions, in particular surfaces, of treatment machine 1 and/or sterilizing and cleaning apparatus 17 and/or active agent conditioning devices 30 are cleaned by means of the cleaning and/or sterilization mixture while treatment machine 1 is controlled into the initial cleaning configuration. In particular, during the step of cleaning, residues, in particular solid residues, originating from the cleaning and/or sterilization mixture are removed.

In use, also a step of providing for the cleaning and/or sterilization mixture is executed, in particular by means of sterilization and cleaning apparatus 17, in particular by each active agent conditioning devices 30 (independently from the other ones).

Advantageously, during the step of sterilizing the temperature of the respective cleaning and/or sterilization mixture is controlled to be above the threshold temperature, in particular above 100°C, even more particular above or equal to 110°C, even most particular above or equal to 130 °C, in particular independently on whether treatment machine 1 is controlled into the production or sterilization configuration; and during the step of cleaning the temperature of the respective cleaning and/or sterilization mixture is controlled to be below the temperature threshold, in particular below 100°C, even more particular below 70°C, in particular when treatment machine 1 is controlled into the cleaning configuration.

Preferentially but not necessarily, during the step of sterilizing the temperature of the respective cleaning and/or sterilization mixture is controlled to be range between 100°C to 200 °C, in particular to range between 110°C and 200 °C, even more particular to range between 130 °C to 200 °C.

According to a preferred non-limiting embodiment, operation of treatment machine 1 comprises at least a step of controlling at least one of the active agent conditioning devices 30 into a sterilizing configuration or a cleaning configuration. In particular, during the step of controlling, each one of the active agent conditioning devices 30 is (selectively and/or independently) controlled into the respective sterilizing configuration or the respective cleaning configuration.

According to a preferred non-limiting embodiment, each active agent conditioning device 30 controls the temperature of the respective cleaning and/or sterilization mixture to be above the temperature threshold, in particular above 100 °C, even more particular to be at least 110 °C, even most particular to be at least 130 °C, when being controlled into the sterilizing configuration and to be below the threshold temperature, in particular below 100 °C, even more particular to be below 70°C, when being controlled into the cleaning configuration.

Preferentially but not necessarily, the temperature of the cleaning and/or sterilization mixture is controlled by means of the respective heating device, in particular the respective evaporator 35. According to a non-limiting embodiment, the respective heating device, in particular the respective evaporator 35 is deactivate as the required and/or desired temperature of the cleaning and/or sterilization mixture is obtainable without heating, in particular with the respective active agent conditioning device 30 being controlled into the cleaning configuration.

Preferentially but not necessarily, each active agent conditioning device 30 is controlled into the sterilization configuration so that the active agent conditioning device 30 conditions the cleaning and/or sterilization mixture during the step of sterilizing and/or into the cleaning configuration during the step of cleaning.

Preferentially but not necessarily, each active agent conditioning device 30 controls the concentration of the hydrogen peroxide of the cleaning and/or sterilization mixture to range between 2000 ppm to 100000 ppm, in particular by mixing the respective initial hydrogen peroxide mixture with the respective carrier gas, in particular the pressurized air, for obtaining the cleaning and/or sterilization mixture.

In particular, the mixing of the respective initial hydrogen peroxide mixture with the carrier gas is controlled by means of the respective mixing assembly 31, in particular the respective valves 32 and 33.

The advantages of treatment machine 1 and the methods according to the present invention will be clear from the foregoing description.

In particular, by controlling the temperature of the cleaning and/or sterilization mixture above or below 100°C it is possible to obtain respectively a sterilization or cleaning effect. This means that the device used for the sterilization can also be used for the cleaning without the need of removing any parts from treatment machine 1. This reduces the downtime when a cleaning of treatment machine 1 is required, meaning that production can be resumed earlier.

Clearly, changes may be made to treatment machine 1 and the method as described herein without, however, departing from the scope of protection as defined in the accompanying claims.

## Claims

1. Treatment machine (1) for executing at least one treatment on a receptacle (2, 3);
the treatment machine (1) comprises at least:
- a control unit (5) configured to control operation of the treatment machine (1); and
- an active agent conditioning device (30) configured to condition a cleaning and/or sterilization mixture comprising at least hydrogen peroxide;
wherein the active agent conditioning device (30) is configured to at least control the temperature of the cleaning and/or sterilization mixture;
wherein the control unit (5) is configured to control the active agent conditioning device (30) into at least:
- a sterilizing configuration; and
- a cleaning configuration;
wherein the active agent conditioning device (30) is configured to control the temperature of the cleaning and/or sterilization mixture to be above a threshold temperature, in particular above 100 °C, even more particular to be at least 110 °C, when being controlled into the sterilizing configuration; and
wherein the active agent conditioning device (30) is configured to control the temperature of the cleaning and/or sterilization mixture to be below the threshold temperature, in particular below 100 °C, even more particular to be below 70°C, when being controlled into the cleaning configuration.

2. Treatment machine according to claim 1, wherein the control unit (5) is configured to control the active agent conditioning device (30) into the sterilization configuration so that, in use, the active agent conditioning device (30) conditions the cleaning and/or sterilization mixture for sterilizing portions of the treatment machine (1) and/or of the receptacles (2, 3) and/or of closures (4) for closing the receptacles (2) and/or of precursors (3) of the receptacles (2).

3. Treatment machine according to claim 1 or 2, wherein the control unit (5) is configured to control the active agent conditioning device (30) into the cleaning configuration so that, in use, the active agent conditioning device (30) conditions the cleaning and/or sterilization mixture for cleaning portions of the treatment machine (1) and/or of the active agent conditioning device (30), in particular for removing residues, even more particular solid residues, originating from the cleaning and/or sterilization mixture.

4. Treatment machine according to any one of the preceding claims, wherein the active agent conditioning device (30) is also configured to condition the cleaning and/or sterilization mixture such that the hydrogen peroxide concentration of the cleaning and sterilization mixture ranges between 2000 ppm to 100000 ppm.

5. Treatment machine according to any one of the preceding claims, wherein the active agent conditioning device (30) comprises a mixing assembly (31) configured to mix an initial hydrogen peroxide mixture with a carrier gas for obtaining the cleaning and/or sterilization mixture and so as to control the hydrogen peroxide concentration of the cleaning and/or sterilization mixture, in particular such that the hydrogen peroxide concentration ranges between 2000 ppm to 100000 ppm.

6. Treatment machine according to any one of the preceding claims, wherein the active agent conditioning device (30) further comprises a heating device, in particular an evaporator (35), for controlling the temperature and/or the physical state of the cleaning and/or sterilization mixture.

7. Treatment machine according to any one of the preceding claims, further comprising at least:
- a filling apparatus (15) configured to fill the receptacles (3) with a pourable product; and/or
- a capping apparatus (16) configured to apply closures (4) onto the receptacles (2); and/or
- a molding apparatus (14) configured to form the receptacles (2) from preforms (3); and/or
- a preform preparation apparatus (13) configured to prepare the preforms (2).

8. Method of operating a treatment machine (1) for the treatment of receptacles (2, 3), the method comprising at least the steps of:
- providing for a cleaning and/or sterilization mixture comprising hydrogen peroxide;
- sterilizing at least portions of the treatment machine (1) and/or at least portions of the receptacles (2, 3) and/or of receptacle closures (4) by means of the cleaning and/or sterilization mixture; and
- cleaning at least portions of the treatment machine (1) by means of the cleaning and/or sterilization mixture, in particular for removing residues, even more particular solid residues, originating from the cleaning and/or sterilization mixture;
wherein during the step of sterilizing the temperature of the cleaning and/or sterilization mixture is controlled to be above a temperature threshold, in particular above 100°C, even more particular above or substantially equal to 110°C; and
wherein during the step of cleaning the temperature of the cleaning and/or sterilization mixture is controlled to be below the threshold temperature, in particular below 100°C, in particular below 70°C.

9. Method according to claim 8, wherein the hydrogen peroxide concentration within the cleaning and/or sterilization mixture ranges between 2000 ppm to 100000 ppm.

10. The method according to claim 8 or 9, further comprising the step of mixing, during which an initial hydrogen peroxide mixture is mixed with a carrier gas for obtaining the cleaning and/or sterilization mixture and so as to control the hydrogen peroxide concentration of the cleaning and/or sterilization mixture, in particular such that the hydrogen peroxide concentration of the cleaning and/or sterilization mixture ranges between 2000 ppm to 100000 ppm.

11. Method according to any one of claims 8 to 10, wherein a heating device, in particular an evaporator (35), controls the temperature and/or the physical state of the cleaning and/or sterilization mixture.

12. Method according to any one of claims 8 to 11, wherein the step of sterilizing is executed during operation of the treatment machine (1) and the step of cleaning is executed during a production stop or prior or after operation of the treatment machine (1).

13. Method of operating a treatment machine for the treatment of receptacles;
the treatment machine comprising at least an active agent conditioning device (30) for conditioning a cleaning and/or sterilization mixture comprising at least hydrogen peroxide;
the method comprising at least the step of controlling the active agent conditioning device (30) into a sterilizing configuration or a cleaning configuration;
wherein the active agent conditioning device (30) controls the temperature of the cleaning and/or sterilization mixture to be above a threshold temperature, in particular above 100 °C, even more particular to be at least 110 °C, when being controlled into the sterilizing configuration; and to be below the threshold temperature, in particular below 100 °C, even more particular to be below 70°C, when being controlled into the cleaning configuration.

14. Method according to claim 13, wherein the active agent conditioning device (30) is controlled into the sterilization configuration so that the active agent conditioning device (30) conditions the cleaning and/or sterilization mixture for sterilizing portions of the treatment machine (1) and/or of the receptacles (2, 3) and/or of closures (4) for closing the receptacles (2) and/or of precursors (3) of the receptacles (2); and/or
the active agent conditioning device (30) is controlled into the cleaning configuration so that the active agent conditioning device (30) conditions the cleaning and/or sterilization mixture for cleaning portions of the treatment machine (1) and/or of the active agent conditioning device (30), in particular for removing residues, even more particular solid residues, originating from the cleaning and/or sterilization mixture.

15. Method according to claim 13 or 14, wherein the active agent conditioning device (30) controls the concentration of the hydrogen peroxide of the cleaning and/or sterilization mixture to range between 2000 ppm to 100000 ppm, in particular by mixing an initial hydrogen peroxide mixture with a gas for obtaining the cleaning and/or sterilization mixture.
